# EUROPEAN PATENT APPLICATION

(11) **EP 0 953 638 A1**
(43) Date of publication of application: **03.11.1999**
(21) Application number: 98400565.2
(22) Date of filing: 11.03.1998
(51) Int. Cl.: C12N 15/12, C07K 14/705, C07K 16/28, A61K 38/17, C12Q 1/68, G01N 33/50, A61K 31/70

(54) **Human vanilloid receptor-like cation channel**

(71) Applicant: SYNTHELABO, 92350 Le Plessis Robinson (FR)
(72) Inventor: Partiseti, Michel, 92160 Antony (FR); Renard, Stéphane, 92500 Rueil Malmaison (FR)
(74) Representative: Thouret-Lemaitre, Elisabeth

(57) **Abstract**

hVRCC polypeptides and polynucleotides and methods for producing such polypeptides by recombinant techniques are disclosed. Also disclosed are methods for utilizing hVRCC polypeptides and polynucleotides in the design of protocols for the treatment of acute and chronic inflammation, acute and chronic pain, brain diseases, abnormal proliferation and cancer, ulcer, autoimmune diseases, to mimic or antagonize effect of endogenous neurotransmitters and hormones, to inhibit graft rejection by promoting immunosuppression, among others and diagnostic assays for such conditions.

SEQUENCE LISTING

(1) GENERAL INFORMATION:

(i) APPLICANT:
(A) NAME: SYNTHELABO
(B) STREET: 22 avenue Galilee
(C) CITY: LE PLESSIS-ROBINSON
(E) COUNTRY: FRANCE
(F) POSTAL CODE (ZIP): 92350
(G) TELEPHONE: (33) 1 45 37 56 76

(ii) TITLE OF INVENTION: Human vanilloid receptor-like cation
channel

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 2783 base pairs
(B) TYPE: nucleic acid
(C) STRANDEDNESS: double
(D) TOPOLOGY: circular

(ii) MOLECULE TYPE: cDNA to mRNA

(iii) HYPOTHETICAL: NO

(iv) ANTI-SENSE: NO

(vi) ORIGINAL SOURCE:
(A) ORGANISM: Homo sapiens

(ix) FEATURE:
(A) NAME/KEY: 5'UTR
(B) LOCATION:1..360

(ix) FEATURE:
(A) NAME/KEY: CDS
(B) LOCATION:361..2649

(ix) FEATURE:
(A) NAME/KEY: 3'UTR
(B) LOCATION:2650..2783

(ix) FEATURE:
(A) NAME/KEY: allele
(B) LOCATION:replace(374, "t")

(ix) FEATURE:
(A) NAME/KEY: allele

(B) LOCATION:replace(750, "g")

(ix) FEATURE:
(A) NAME/KEY: allele
(B) LOCATION:replace(787, "c")

(ix) FEATURE:
(A) NAME/KEY: allele
(B) LOCATION:replace(1612, "cagg")
(D) OTHER INFORMATION:/label= GLUTAMINE

(xi) SEQUENCE DESCRIPTION: SEQ ID NO: 1:

GGCTAGCCTG TCCTGACAGG GGAGAGTTAA GCTCCCGTTC TCCACCGTGC CGGCTGGCCA
60

GGTGGGCTGA GGGTGACCGA GAGACCAGAA CCTGCTTGCT GGAGCTTAGT GCTCAGAGCT
120

GGGGAGGGAG GTTCCGCCGC TCCTCTGCTG TCAGCGCCGG CAGCCCCTCC CGGCTTCACT
180

TCCTCCCGCA GCCCCTGCTA CTGAGAAGCT CCGGGATCCC AGCAGCCGCC ACGCCCTGGC
240

CTCAGCCTGC GGGGCTCCAG TCAGGCCAAC ACCGACGCGC AGCTGGGAGG AAGACAGGAC
300

CCTTGACATC TCCATCTGCA CAGAGGTCCT GGCTGGACCG AGCAGCCTCC TCCTCCTAGG
360

ATG ACC TCA CCC TCC AGC TCT CCA GTT TTC AGG TTG GAG ACA TTA GAT
408
Met Thr Ser Pro Ser Ser Ser Pro Val Phe Arg Leu Glu Thr Leu Asp
1               5                   10                  15

GGA GGC CAA GAA GAT GGC TCT GAG GCG GAC AGA GGA AAG CTG GAT TTT
456
Gly Gly Gln Glu Asp Gly Ser Glu Ala Asp Arg Gly Lys Leu Asp Phe
            20                  25                  30

GGG AGC GGG CTG CCT CCC ATG GAG TCA CAG TTC CAG GGC GAG GAC CGG
504
Gly Ser Gly Leu Pro Pro Met Glu Ser Gln Phe Gln Gly Glu Asp Arg
        35                  40                  45

AAA TTC GCC CCT CAG ATA AGA GTC AAC CTC AAC TAC CGA AAG GGA ACA
552
Lys Phe Ala Pro Gln Ile Arg Val Asn Leu Asn Tyr Arg Lys Gly Thr
    50                  55                  60

GGT GCC AGT CAG CCG GAT CCA AAC CGA TTT GAC CGA GAT CGG CTC TTC
600
Gly Ala Ser Gln Pro Asp Pro Asn Arg Phe Asp Arg Asp Arg Leu Phe
65                  70                  75                  80

AAT GCG GTC TCC CGG GGT GTC CCC GAG GAT CTG GCT GGA CTT CCA GAG
648

Asn Ala Val Ser Arg Gly Val Pro Glu Asp Leu Ala Gly Leu Pro Glu
                85                  90                  95

TAC CTG AGC AAG ACC AGC AAG TAC CTC ACC GAC TCG GAA TAC ACA GAG
696
Tyr Leu Ser Lys Thr Ser Lys Tyr Leu Thr Asp Ser Glu Tyr Thr Glu
            100                 105                 110

GGC TCC ACA GGT AAG ACG TGC CTG ATG AAG GCT GTG CTG AAC CTT AAG
744
Gly Ser Thr Gly Lys Thr Cys Leu Met Lys Ala Val Leu Asn Leu Lys
        115                 120                 125

GAC GGA GTC AAT GCC TGC ATT CTG CCA CTG CTG CAG ATC GAC AGG GAC
792
Asp Gly Val Asn Ala Cys Ile Leu Pro Leu Leu Gln Ile Asp Arg Asp
    130                 135                 140

TCT GGC AAT CCT CAG CCC CTG GTA AAT GCC CAG TGC ACA GAT GAC TAT
840
Ser Gly Asn Pro Gln Pro Leu Val Asn Ala Gln Cys Thr Asp Asp Tyr
145                 150                 155                 160

TAC CGA GGC CAC AGC GCT CTG CAC ATC GCC ATT GAG AAG AGG AGT CTG
888
Tyr Arg Gly His Ser Ala Leu His Ile Ala Ile Glu Lys Arg Ser Leu
                165                 170                 175

CAG TGT GTG AAG CTC CTG GTG GAG AAT GGG GCC AAT GTG CAT GCC CGG
936
Gln Cys Val Lys Leu Leu Val Glu Asn Gly Ala Asn Val His Ala Arg
            180                 185                 190

GCC TGC GGC CGC TTC TTC CAG AAG GGC CAA GGG ACT TGC TTT TAT TTC
984
Ala Cys Gly Arg Phe Phe Gln Lys Gly Gln Gly Thr Cys Phe Tyr Phe
        195                 200                 205

GGT GAG CTA CCC CTC TCT TTG GCC GCT TGC ACC AAG CAG TGG GAT GTG
1032
Gly Glu Leu Pro Leu Ser Leu Ala Ala Cys Thr Lys Gln Trp Asp Val
    210                 215                 220

GTA AGC TAC CTC CTG GAG AAC CCA CAC CAG CCC GCC AGC CTG CAG GCC
1080
Val Ser Tyr Leu Leu Glu Asn Pro His Gln Pro Ala Ser Leu Gln Ala
225                 230                 235                 240

ACT GAC TCC CAG GGC AAC ACA GTC CTG CAT GCC CTA GTG ATG ATC TCG
1128
Thr Asp Ser Gln Gly Asn Thr Val Leu His Ala Leu Val Met Ile Ser
                245                 250                 255

GAC AAC TCA GCT GAG AAC ATT GCA CTG GTG ACC AGC ATG TAT GAT GGG
1176
Asp Asn Ser Ala Glu Asn Ile Ala Leu Val Thr Ser Met Tyr Asp Gly
            260                 265                 270

CTC CTC CAA GCT GGG GCC CGC CTC TGC CCT ACC GTG CAG CTT GAG GAC
1224

Leu Leu Gln Ala Gly Ala Arg Leu Cys Pro Thr Val Gln Leu Glu Asp
        275                 280                 285

ATC CGC AAC CTG CAG GAT CTC ACG CCT CTG AAG CTG GCC GCC AAG GAG
1272
Ile Arg Asn Leu Gln Asp Leu Thr Pro Leu Lys Leu Ala Ala Lys Glu
    290                 295                 300

GGC AAG ATC GAG ATT TTC AGG CAC ATC CTG CAG CGG GAG TTT TCA GGA
1320
Gly Lys Ile Glu Ile Phe Arg His Ile Leu Gln Arg Glu Phe Ser Gly
305                 310                 315                 320

CTG AGC CAC CTT TCC CGA AAG TTC ACC GAG TGG TGC TAT GGG CCT GTC
1368
Leu Ser His Leu Ser Arg Lys Phe Thr Glu Trp Cys Tyr Gly Pro Val
                325                 330                 335

CGG GTG TCG CTG TAT GAC CTG GCT TCT GTG GAC AGC TGT GAG GAG AAC
1416
Arg Val Ser Leu Tyr Asp Leu Ala Ser Val Asp Ser Cys Glu Glu Asn
            340                 345                 350

TCA GTG CTG GAG ATC ATT GCC TTT CAT TGC AAG AGC CCG CAC CGA CAC
1464
Ser Val Leu Glu Ile Ile Ala Phe His Cys Lys Ser Pro His Arg His
        355                 360                 365

CGA ATG GTC GTT TTG GAG CCC CTG AAC AAA CTG CTG CAG GCG AAA TGG
1512
Arg Met Val Val Leu Glu Pro Leu Asn Lys Leu Leu Gln Ala Lys Trp
    370                 375                 380

GAT CTG CTC ATC CCC AAG TTC TTC TTA AAC TTC CTG TGT AAT CTG ATC
1560
Asp Leu Leu Ile Pro Lys Phe Phe Leu Asn Phe Leu Cys Asn Leu Ile
385                 390                 395                 400

TAC ATG TTC ATC TTC ACC GCT GTT GCC TAC CAT CAG CCT ACC CTG AAG
1608
Tyr Met Phe Ile Phe Thr Ala Val Ala Tyr His Gln Pro Thr Leu Lys
                405                 410                 415

AAG GCC GCC CCT CAC CTG AAA GCG GAG GTT GGA AAC TCC ATG CTG CTG
1656
Lys Ala Ala Pro His Leu Lys Ala Glu Val Gly Asn Ser Met Leu Leu
            420                 425                 430

ACG GGC CAC ATC CTT ATC CTG CTA GGG GGG ATC TAC CTC CTC GTG GGC
1704
Thr Gly His Ile Leu Ile Leu Leu Gly Gly Ile Tyr Leu Leu Val Gly
        435                 440                 445

CAG CTG TGG TAC TTC TGG CGG CGC CAC GTG TTC ATC TGG ATC TCG TTC
1752
Gln Leu Trp Tyr Phe Trp Arg Arg His Val Phe Ile Trp Ile Ser Phe
    450                 455                 460

ATA GAC AGC TAC TTT GAA ATC CTC TTC CTG TTC CAG GCC CTG CTC ACA
1800

Ile Asp Ser Tyr Phe Glu Ile Leu Phe Leu Phe Gln Ala Leu Leu Thr
465                 470                 475                 480

GTG GTG TCC CAG GTG CTG TGT TTC CTG GCC ATC GAG TGG TAC CTG CCC
1848
Val Val Ser Gln Val Leu Cys Phe Leu Ala Ile Glu Trp Tyr Leu Pro
                485                 490                 495

CTG CTT GTG TCT GCG CTG GTG CTG GGC TGG CTG AAC CTG CTT TAC TAT
1896
Leu Leu Val Ser Ala Leu Val Leu Gly Trp Leu Asn Leu Leu Tyr Tyr
            500                 505                 510

ACA CGT GGC TTC CAG CAC ACA GGC ATC TAC AGT GTC ATG ATC CAG AAG
1944
Thr Arg Gly Phe Gln His Thr Gly Ile Tyr Ser Val Met Ile Gln Lys
        515                 520                 525

GTC ATC CTG CGG GAC CTG CTG CGC TTC CTT CTG ATC TAC TTA GTC TTC
1992
Val Ile Leu Arg Asp Leu Leu Arg Phe Leu Leu Ile Tyr Leu Val Phe
    530                 535                 540

CTT TTC GGC TTC GCT GTA GCC CTG GTG AGC CTG AGC CAG GAG GCT TGG
2040
Leu Phe Gly Phe Ala Val Ala Leu Val Ser Leu Ser Gln Glu Ala Trp
545                 550                 555                 560

CGC CCC GAA GCT CCT ACA GGC CCC AAT GCC ACA GAG TCA GTG CAG CCC
2088
Arg Pro Glu Ala Pro Thr Gly Pro Asn Ala Thr Glu Ser Val Gln Pro
                565                 570                 575

ATG GAG GGA CAG GAG GAC GAG GGC AAC GGG GCC CAG TAC AGG GGT ATC
2136
Met Glu Gly Gln Glu Asp Glu Gly Asn Gly Ala Gln Tyr Arg Gly Ile
            580                 585                 590

CTG GAA GCC TCC TTG GAG CTC TTC AAA TTC ACC ATC GGC ATG GGC GAG
2184
Leu Glu Ala Ser Leu Glu Leu Phe Lys Phe Thr Ile Gly Met Gly Glu
        595                 600                 605

CTG GCC TTC CAG GAG CAG CTG CAC TTC CGC GGC ATG GTG CTG CTG CTG
2232
Leu Ala Phe Gln Glu Gln Leu His Phe Arg Gly Met Val Leu Leu Leu
    610                 615                 620

CTG CTG GCC TAC GTG CTG CTC ACC TAC ATC CTG CTG CTC AAC ATG CTC
2280
Leu Leu Ala Tyr Val Leu Leu Thr Tyr Ile Leu Leu Leu Asn Met Leu
625                 630                 635                 640

ATC GCC CTC ATG AGC GAG ACC GTC AAC AGT GTC GCC ACT GAC AGC TGG
2328
Ile Ala Leu Met Ser Glu Thr Val Asn Ser Val Ala Thr Asp Ser Trp
                645                 650                 655

AGC ATC TGG AAG CTG CAG AAA GCC ATC TCT GTC CTG GAG ATG GAG AAT
2376

Ser Ile Trp Lys Leu Gln Lys Ala Ile Ser Val Leu Glu Met Glu Asn
            660                 665                 670

GGC TAT TGG TGG TGC AGG AAG AAG CAG CGG GCA GGT GTG ATG CTG ACC
2424
Gly Tyr Trp Trp Cys Arg Lys Lys Gln Arg Ala Gly Val Met Leu Thr
        675                 680                 685

GTT GGC ACT AAG CCA GAT GGC AGC CCC GAT GAG CGC TGG TGC TTC AGG
2472
Val Gly Thr Lys Pro Asp Gly Ser Pro Asp Glu Arg Trp Cys Phe Arg
    690                 695                 700

GTG GAG GAG GTG AAC TGG GCT TCA TGG GAG CAG ACG CTG CCT ACG CTG
2520
Val Glu Glu Val Asn Trp Ala Ser Trp Glu Gln Thr Leu Pro Thr Leu
705                 710                 715                 720

TGT GAG GAC CCG TCA GGG GCA GGT GTC CCT CGA ACT CTC GAG AAC CCT
2568
Cys Glu Asp Pro Ser Gly Ala Gly Val Pro Arg Thr Leu Glu Asn Pro
                725                 730                 735

GTC CTG GCT TCC CCT CCC AAG GAG GAT GAG GAT GGT GCC TCT GAG GAA
2616
Val Leu Ala Ser Pro Pro Lys Glu Asp Glu Asp Gly Ala Ser Glu Glu
            740                 745                 750

AAC TAT GTG CCC GTC CAG CTC CTC CAG TCC AAC TGATGGCCCA GATGCAGCAG
2669
Asn Tyr Val Pro Val Gln Leu Leu Gln Ser Asn
        755                 760

GAGGCCAGAG GACAGAGCAG AGGATCTTTC CAACCACATC TGCTGGCTCT GGGGTCCCAG
2729

TGAATTCTGG TGGCAAATAT ATATTTTCAC TAACTAAAAA AAAAAAAAAA AAAA
2783


(2) INFORMATION FOR SEQ ID NO: 2:

        (i) SEQUENCE CHARACTERISTICS:
            (A) LENGTH: 763 amino acids
            (B) TYPE: amino acid
            (D) TOPOLOGY: linear

    (ii) MOLECULE TYPE: protein
    (xi) SEQUENCE DESCRIPTION: SEQ ID NO: 2:

Met Thr Ser Pro Ser Ser Ser Pro Val Phe Arg Leu Glu Thr Leu Asp
  1               5                  10                  15

Gly Gly Gln Glu Asp Gly Ser Glu Ala Asp Arg Gly Lys Leu Asp Phe
             20                  25                  30

Gly Ser Gly Leu Pro Pro Met Glu Ser Gln Phe Gln Gly Glu Asp Arg
         35                  40                  45

Lys Phe Ala Pro Gln Ile Arg Val Asn Leu Asn Tyr Arg Lys Gly Thr
    50                  55                  60

Gly Ala Ser Gln Pro Asp Pro Asn Arg Phe Asp Arg Asp Arg Leu Phe
65                  70                  75                  80

Asn Ala Val Ser Arg Gly Val Pro Glu Asp Leu Ala Gly Leu Pro Glu
                85                  90                  95

Tyr Leu Ser Lys Thr Ser Lys Tyr Leu Thr Asp Ser Glu Tyr Thr Glu
            100                 105                 110

Gly Ser Thr Gly Lys Thr Cys Leu Met Lys Ala Val Leu Asn Leu Lys
        115                 120                 125

Asp Gly Val Asn Ala Cys Ile Leu Pro Leu Leu Gln Ile Asp Arg Asp
    130                 135                 140

Ser Gly Asn Pro Gln Pro Leu Val Asn Ala Gln Cys Thr Asp Asp Tyr
145                 150                 155                 160

Tyr Arg Gly His Ser Ala Leu His Ile Ala Ile Glu Lys Arg Ser Leu
                165                 170                 175

Gln Cys Val Lys Leu Leu Val Glu Asn Gly Ala Asn Val His Ala Arg
            180                 185                 190

Ala Cys Gly Arg Phe Phe Gln Lys Gly Gln Gly Thr Cys Phe Tyr Phe
        195                 200                 205

Gly Glu Leu Pro Leu Ser Leu Ala Ala Cys Thr Lys Gln Trp Asp Val
    210                 215                 220

Val Ser Tyr Leu Leu Glu Asn Pro His Gln Pro Ala Ser Leu Gln Ala
225                 230                 235                 240

Thr Asp Ser Gln Gly Asn Thr Val Leu His Ala Leu Val Met Ile Ser
                245                 250                 255

Asp Asn Ser Ala Glu Asn Ile Ala Leu Val Thr Ser Met Tyr Asp Gly
            260                 265                 270

Leu Leu Gln Ala Gly Ala Arg Leu Cys Pro Thr Val Gln Leu Glu Asp
        275                 280                 285

Ile Arg Asn Leu Gln Asp Leu Thr Pro Leu Lys Leu Ala Ala Lys Glu
    290                 295                 300

Gly Lys Ile Glu Ile Phe Arg His Ile Leu Gln Arg Glu Phe Ser Gly
305                 310                 315                 320

Leu Ser His Leu Ser Arg Lys Phe Thr Glu Trp Cys Tyr Gly Pro Val
                325                 330                 335

Arg Val Ser Leu Tyr Asp Leu Ala Ser Val Asp Ser Cys Glu Glu Asn
            340                 345                 350

Ser Val Leu Glu Ile Ile Ala Phe His Cys Lys Ser Pro His Arg His
        355                 360                 365

Arg Met Val Val Leu Glu Pro Leu Asn Lys Leu Leu Gln Ala Lys Trp
    370                 375                 380

Asp Leu Leu Ile Pro Lys Phe Phe Leu Asn Phe Leu Cys Asn Leu Ile
385                 390                 395                 400

Tyr Met Phe Ile Phe Thr Ala Val Ala Tyr His Gln Pro Thr Leu Lys
                405                 410                 415

Lys Ala Ala Pro His Leu Lys Ala Glu Val Gly Asn Ser Met Leu Leu
            420                 425                 430

Thr Gly His Ile Leu Ile Leu Leu Gly Gly Ile Tyr Leu Leu Val Gly
        435                 440                 445

Gln Leu Trp Tyr Phe Trp Arg Arg His Val Phe Ile Trp Ile Ser Phe
    450                 455                 460

Ile Asp Ser Tyr Phe Glu Ile Leu Phe Leu Phe Gln Ala Leu Leu Thr
465                 470                 475                 480

Val Val Ser Gln Val Leu Cys Phe Leu Ala Ile Glu Trp Tyr Leu Pro
                485                 490                 495

Leu Leu Val Ser Ala Leu Val Leu Gly Trp Leu Asn Leu Leu Tyr Tyr
            500                 505                 510

Thr Arg Gly Phe Gln His Thr Gly Ile Tyr Ser Val Met Ile Gln Lys
        515                 520                 525

Val Ile Leu Arg Asp Leu Leu Arg Phe Leu Leu Ile Tyr Leu Val Phe
    530                 535                 540

Leu Phe Gly Phe Ala Val Ala Leu Val Ser Leu Ser Gln Glu Ala Trp
545                 550                 555                 560

Arg Pro Glu Ala Pro Thr Gly Pro Asn Ala Thr Glu Ser Val Gln Pro
                565                 570                 575

Met Glu Gly Gln Glu Asp Glu Gly Asn Gly Ala Gln Tyr Arg Gly Ile
            580                 585                 590

Leu Glu Ala Ser Leu Glu Leu Phe Lys Phe Thr Ile Gly Met Gly Glu
        595                 600                 605

Leu Ala Phe Gln Glu Gln Leu His Phe Arg Gly Met Val Leu Leu Leu
    610                 615                 620

Leu Leu Ala Tyr Val Leu Leu Thr Tyr Ile Leu Leu Leu Asn Met Leu
625                 630                 635                 640

Ile Ala Leu Met Ser Glu Thr Val Asn Ser Val Ala Thr Asp Ser Trp
                645                 650                 655

Ser Ile Trp Lys Leu Gln Lys Ala Ile Ser Val Leu Glu Met Glu Asn
            660                 665                 670

Gly Tyr Trp Trp Cys Arg Lys Lys Gln Arg Ala Gly Val Met Leu Thr
        675                 680                 685

Val Gly Thr Lys Pro Asp Gly Ser Pro Asp Glu Arg Trp Cys Phe Arg
    690                 695                 700

Val Glu Glu Val Asn Trp Ala Ser Trp Glu Gln Thr Leu Pro Thr Leu
705                 710                 715                 720

Cys Glu Asp Pro Ser Gly Ala Gly Val Pro Arg Thr Leu Glu Asn Pro
                725                 730                 735

Val Leu Ala Ser Pro Pro Lys Glu Asp Glu Asp Gly Ala Ser Glu Glu
            740                 745                 750

Asn Tyr Val Pro Val Gln Leu Leu Gln Ser Asn
        755                 760

## Description

### FIELD OF INVENTION

This invention relates to newly identified polynucleotides, polypeptides encoded by such polynucleotides, to the use of such polynucleotides and polypeptides and to their production. More particularly, the polynucleotides and polypeptides of the present invention relate to vanilloid receptor-like channel family, hereinafter referred to as hVRCC (**H**uman **V**anilloid **R**eceptor-like **C**ation **C**hannel). The invention also relates to inhibiting or activating the action of such polynucleotides and polypeptides.

### BACKGROUND OF THE INVENTION

Vanilloids are natural compounds which are known to trigger cation permeability in subpopulations of peripheral neurons. These ones, also called "nociceptors", are involved in physiological processes such as transmission to the central nervous system of noxious stimuli, said stimuli being mechanical, chemical or thermal (Jessel and Kelly, 1991, pp 385-399, Principal of Neural Sciences, third edition, edited by Kandel et al.). Recently, a new cation channel was discovered and isolated from rat (Caterina et al., 1997, Nature 389 pp 816-824). This channel is activated by vanilloids such as capsaicin and resiniferatoxin and is highly expressed in adult dorsal root ganglia. This channel has also been shown to have significant structural similarities with the "store-operated" calcium channel family i. e., six putative transmembrane domains . A major functional characteristic of this capsaicin-gated conductance is that it is highly selective for the divalent cation calcium even if it is also permeant to magnesium and monovalent cations such as sodium, potassium and cesium. This indicates that these channels have an interesting potential as therapeutic targets. Clearly there is a need for identification and characterization of further channels which can play a role in (i) preventing, ameliorating or correcting dysfunctions or diseases, including, but not limited to, acute and chronic inflammation, acute and chronic pain, brain diseases, ulcer, abnormal proliferation and cancer, autoimmune diseases and (ii) mimicking or antagonizing effect of endogenous neurotransmitters and hormones and inhibiting graft rejection by promoting immunosuppression.

### SUMMARY OF THE INVENTION

In one aspect, the invention relates to hVRCC polypeptides and recombinant materials and methods for their production. Another aspect of the invention relates to methods for using such hVRCC polypeptides and polynucleotides. Such uses include the treatment of acute and chronic inflammation, acute and chronic pain, brain diseases, abnormal proliferation and cancer, ulcer, to mimic or antagonize effect of endogenous neurotransmitters and hormones, or to diagnose or treat any disorder related to abnormal expression of these hVRCC polypeptides, among others. In still another aspect, the invention relates to methods to identify agonists and antagonists using the materials provided by the invention, and treating conditions associated with hVRCC imbalance with the identified compounds. Yet another aspect of the invention relates to diagnostic assays for detecting diseases associated with inappropriate hVRCC activity or levels.

### DESCRIPTION OF THE INVENTION

### Definitions

The following definitions are provided to facilitate understanding of certain terms used frequently herein-below.

"hVRCC" refers, among others, to a polypeptide comprising the amino acid sequence set forth in SEQ ID NO:2, or an allelic variant thereof.

"Receptor Activity" or "Channel Activity" or "Biological Activity of the Receptor" or "Biological Activity of the Channel" refers to the metabolic or physiologic function of said hVRCC including similar activities or improved activities or these activities with decreased undesirable side-effects. Also included are antigenic and immunogenic activities of said hVRCC.

"hVRCC gene" refers to a polynucleotide comprising the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of a Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., "Analysis for protein modifications and nonprotein cofactors", *Meth Enzymol* (1990) 182:626-646 and Rattan *et al*., "Protein Synthesis: Posttranslational Modifications and Aging", *Ann NY Acad Sci (*1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCG program package (Devereux, J., *et al*., *Nucleic Acids Research* (1984) 12(1):387), BLASTP, BLASTN, FASTA (Atschul, S.F. *et al*., *J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to hVRCC polypeptides. The hVRCC polypeptides include the polypeptide of SEQ ID NO:2, as well as polypeptides comprising the amino acid sequence of SEQ ID NO:2 , and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within hVRCC polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% are highly preferred. Preferably hVRCC polypeptides exhibit at least one biological activity of the receptor.

The hVRCC polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the hVRCC polypeptides are also included in the invention. A fragment is a polypeptide having an amino acid sequence that entirely is the same as part, but not all, of the amino acid sequence of the aforementioned hVRCC polypeptides. As with hVRCC polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of hVRCC polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of hVRCC polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate receptor or channel activity, including those with a similar activity or an improved activity, or with a decreased undesirable activity. Also included are those that are antigenic or immunogenic in an animal, especially in a human.

Preferably, all of these polypeptide fragments retain the biological activity of the receptor, including antigenic activity. Variants of the defined sequence and fragments also form part of the present invention. Preferred variants are those that vary from the referents by conservative amino acid substitutions i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The hVRCC polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to hVRCC polynucleotides. hVRCC polynucleotides include isolated polynucleotides which encode the hVRCC polypeptides and fragments, and polynucleotides closely related thereto. More specifically, hVRCC polynucleotides of the invention include a polynucleotide comprising the nucleotide sequence set forth in SEQ ID NO:1 encoding a hVRCC polypeptide of SEQ ID NO: 2, and polynucleotide having the particular sequence of SEQ ID NO:1. hVRCC polynucleotides further include a polynucleotide comprising a nucleotide sequence that has at least 80% identity to a nucleotide sequence encoding the hVRCC polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under hVRCC polynucleotides are a nucleotide sequence which has sufficient identity to a nucleotide sequence contained in SEQ ID NO:1 or contained in the cDNA insert in the plasmid deposited with the ATCC Deposit number 209625 to hybridize under conditions useable for amplification or for use as a probe or marker. Moreover, hVRCC polynucleotide includes nucleotide sequences having at least 80% identity to a nucleotide sequence encoding the hVRCC polypeptide expressed by the cDNA insert deposited at the ATCC with Deposit Number 209625, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above hVRCC polynucleotides.

A deposit containing a human hVRCC cDNA has been deposited with the American Type Culture Collection (ATCC), 12301 Park Lawn Drive, Rockville, Maryland 20852, USA, on February 10, 1998, and assigned ATCC Deposit Number 209625. The deposited material (clone) is a DH5-a strain containing the expression vector bluescript (pBS-SK, Stratagene) that further contains the hVRCC cDNA, referred to as "hVRCC" upon deposit. The cDNA insert is within EcoRI-XhoI site(s) in the vector. The nucleotide sequence of the polynucleotides contained in the deposited material, as well as the amino acid sequence of the polypeptide encoded thereby, are controlling in the event of any conflict with any description of sequences herein. The deposit has been made under the terms of the Budapest Treaty on the international recognition of the deposit of micro-organisms for purposes of patent procedure. The strain will be irrevocably and without restriction or condition released to the public upon the issuance of patent.

hVRCC of the invention is structurally related to other proteins of the store-operated calcium channel family, as shown by the results of sequencing the cDNA of Table 1 (SEQ ID NO:1) encoding human hVRCC. The cDNA sequence of SEQ ID NO:1 contains an open reading frame (nucleotide number 402 to 2696) encoding a polypeptide of 763 amino acids of SEQ ID NO:2. Amino acid sequence of Table 2 (SEQ ID NO:2) has about 49.6%)identity (using blastP version 1.4, GCG program package) in 681 amino acid residues with the rat vanilloid receptor subtype 1 (VR1) protein (accession number: AF029310, Caterina et al., 1997, Nature 389, pp816-824). Nucleotide sequence of Table 1 (SEQ ID NO:1) has about 61% identity (using blastN version 1.4, GCG program package) in 2069 (166 to 2235) nucleotide residues with the VR1 cDNA (accession number: AF029310).

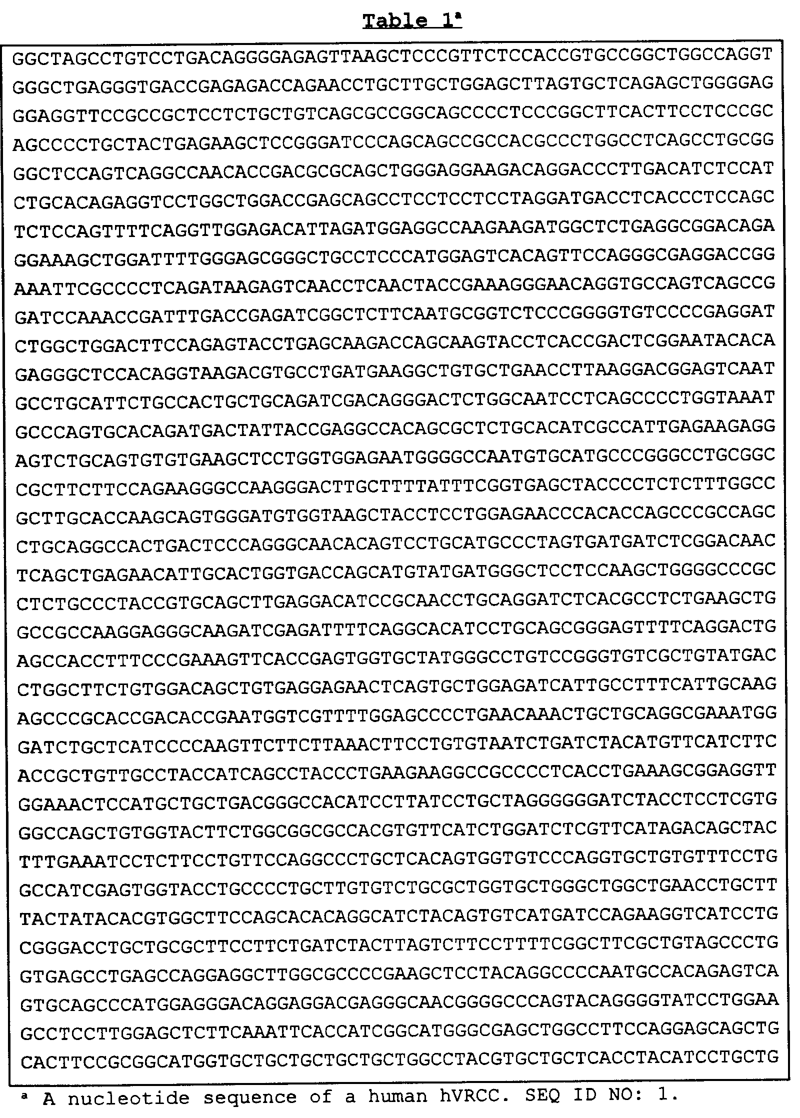

Allelic variants of this sequence have been identified such as a t in position 374, a g in position 750, a c in position 787, and an agg insertion after position 1612, resulting in a glutamine amino-acid insertion in the corresponding position of the protein.

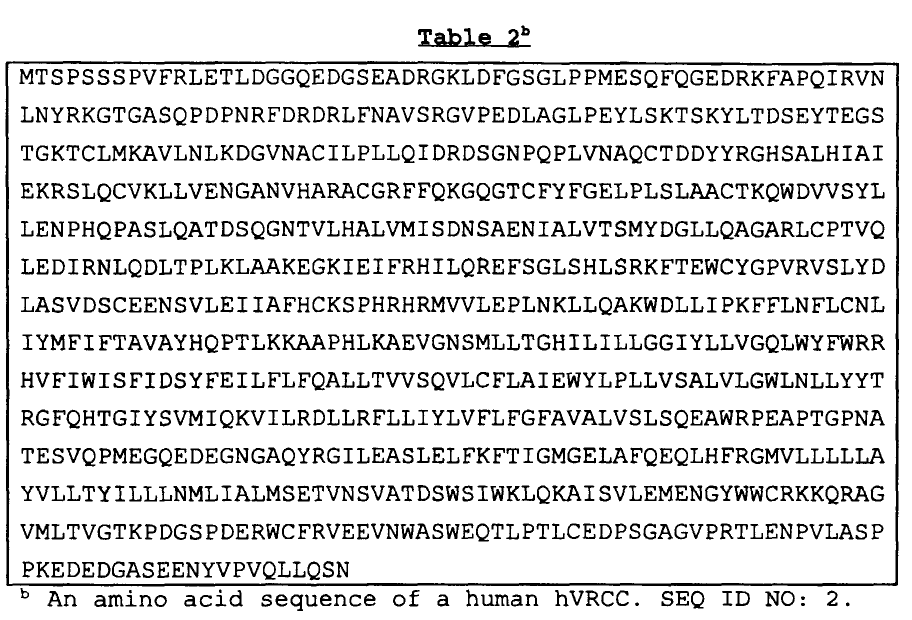

One polynucleotide of the present invention encoding hVRCC may be obtained using standard cloning and screening, from a cDNA library derived from mRNA in macrophages using the expressed sequence tag (EST) analysis (Adams, M.D., *et al. Science* (1991) 252:1651-1656; Adams, M.D. *et al.*, *Nature,* (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding hVRCC polypeptide of SEQ ID NO:2 may be identical to the polypeptide encoding sequence contained in Table 1 (nucleotide number 361 to 2649 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of hVRCC polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al.*, *Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding hVRCC variants comprising the amino acid sequence of hVRCC polypeptide of Table 2 (SEQ ID NO:2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number 209625 or a fragment thereof, may be used as hybridization probes for cDNA and genomic DNA, to isolate full-length cDNAs and genomic clones encoding hVRCC and to isolate cDNA and genomic clones of other genes that have a high sequence similarity to the hVRCC gene. Such hybridisation techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding hVRCC polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces and Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al*., *MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the hVRCC polypeptide is to be expressed for use in screening assays, generally, it is preferred that the polypeptide be produced at the surface of the cell. In this event, the cells may be harvested prior to use in the screening assay. If hVRCC polypeptide is secreted into the medium, the medium can be recovered in order to recover and purify the polypeptide; if produced intracellularly, the cells must first be lysed before the polypeptide is recovered.

hVRCC polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of hVRCC polynucleotides for use as diagnostic reagents. Detection of a mutated form of hVRCC gene associated with a dysfunction will provide a diagnostic tool that can add to or define a diagnosis of a disease or susceptibility to a disease which results from under-expression, over-expression or altered expression of hVRCC. Individuals carrying mutations in the hVRCC gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled hVRCC nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising hVRCC nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to inflammation and pain (both acute and chronic), brain diseases, abnormal proliferation and cancer, autoimmune diseases, through detection of mutation in the hVRCC gene by the methods described.

In addition, inflammation and pain (both acute and chronic), brain diseases, abnormal proliferation and cancer, can be diagnosed by methods comprising determining from a sample derived from a subject an abnormally decreased or increased level of hVRCC polypeptide or hVRCC mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as a hVRCC, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

### Antibodies

The polypeptides of the invention or their fragments or analogs thereof, or cells expressing them can also be used as immunogens to produce antibodies immunospecific for the hVRCC polypeptides. The term "immunospecific" means that the antibodies have substantial greater affinity for the polypeptides of the invention than their affinity for other related polypeptides in the prior art.

Antibodies generated against the hVRCC polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al*., MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against hVRCC polypeptides may also be employed to treat cerebral and cardiac and renal ischemias, brain and cardiac diseases, inflammation, pain, to mimic or antagonize effect of endogenous neurotransmitters and hormones, or to diagnose or treat any disorder related to abnormal expression of said hVRCC polypeptides, among others.

### Vaccines and immunological products

Another aspect of the invention relates to a method for inducing an immunological response in a mammal which comprises inoculating the mammal with hVRCC polypeptide, or a fragment thereof, adequate to produce antibody and/or T cell immune response to protect said animal from inflammation and pain (both acute and chronic),brain diseases, ulcer, abnormal proliferation and cancer, autoimmune diseases, to mimic or antagonize effect of endogenous neurotransmitters and hormones, or to diagnose or treat any disorder related to abnormal expression of the hVRCC polypeptide, among others. Yet another aspect of the invention relates to a method of inducing immunological response in a mammal which comprises, delivering hVRCC polypeptide via a vector directing expression of hVRCC polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a hVRCC polypeptide wherein the composition comprises a hVRCC polypeptide or hVRCC gene. The vaccine formulation may further comprise a suitable carrier. Since hVRCC polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The hVRCC polypeptide of the present invention may be employed in a screening process for compounds which bind the channel and which activate (agonists) or inhibit activation of (antagonists) the channel polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al*., *Current Protocols in Immunology* 1(2):Chapter 5 (1991).

hVRCC polypeptides are implicated in many biological functions, and possibly pathologies. Accordingly, it is desirous to find compounds and drugs which stimulate hVRCC on the one hand and which can inhibit the function of hVRCC on the other hand. In general, agonists are employed for therapeutic and prophylactic purposes for such conditions as to mimic effect of endogenous neurotransmitters and hormones. Antagonists may be employed for a variety of therapeutic and prophylactic purposes for such conditions as inflammation and pain (both acute and chronic), brain diseases, ulcer, abnormal proliferation and cancer, autoimmune diseases, to antagonize effect of endogenous neurotransmitters and hormones and to inhibit graft rejection by promoting immunosuppression.

In general, such screening procedures involve producing appropriate cells which express the receptor polypeptide of the present invention on the surface thereof. Such cells include cells from mammals, yeast, *Drosophila* or *E. coli.* Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the channel is detected by means of a label directly or indirectly associated with the candidate compound or in an assay involving competition with a labeled competitor. Further, these assays may test whether the candidate compound results in a signal generated by activation of the channel, using detection systems appropriate to the cells bearing the channel at their surfaces. Inhibitors of activation are generally assayed in the presence of a known agonist and the effect on activation by the agonist by the presence of the candidate compound is observed. Standard methods for conducting such screening assays are well understood in the art.

The recording of hVRCC channel activity may be carried out either by membrane voltage analysis of transfected cells or microinjected xenope oocytes, directly (patch-clamp for example) or indirectly (fluorescent probes sensitive to changes of intracellular free calcium concentration such as fura-2 and calcium green, Molecular Probes). The assays may simply test binding of a candidate compound wherein adherence to the cells bearing the channel is detected by means of a label directly or indirectly associated with the candidate compound or in an assay involving competition with a labeled competitor. Further, these assays may test whether the candidate compound results in a signal generated by activation of the channel, using detection systems appropriate to the cells bearing the channel at their surfaces. Inhibitors of activation are generally assayed in the presence of a known agonist and the effect on activation by the agonist by the presence of the candidate compound is observed. Standard methods for conducting such screening assays are well understood in the art.

Examples of potential hVRCC antagonists include antibodies or, in some cases, oligonucleotides or proteins which are closely related to the ligand of the hVRCC, e.g., a fragment of the ligand, or small molecules which bind to the receptor but do not elicit a response, so that the activity of the channel is prevented.

### Prophylactic and Therapeutic Methods

This invention provides methods of treating abnormal conditions related to both an excess of and insufficient amounts of hVRCC activity.

If the activity of hVRCC is in excess, several approaches are available. One approach comprises administering to a subject an inhibitor compound (antagonist) as hereinabove described along with a pharmaceutically acceptable carrier in an amount effective to inhibit activation by blocking binding of ligands to the hVRCC, or by inhibiting a second signal, and thereby alleviating the abnormal condition.

In another approach, soluble forms of hVRCC polypeptides still capable of binding the ligand in competition with endogenous hVRCC may be administered. Typical embodiments of such competitors comprise fragments of the hVRCC polypeptide.

In still another approach, expression of the gene encoding endogenous hVRCC can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al*., *Nucleic Acids Res* (1979) 6:3073; Cooney *et al.*, *Science* (1988) 241:456; Dervan *et al*., *Science* (1991) 251:1360. These oligomers can be administered *per se* or the relevant oligomers can be expressed *in vivo.*

For treating abnormal conditions related to an under-expression of hVRCC and its activity, several approaches are also available. One approach comprises administering to a subject a therapeutically effective amount of a compound which activates hVRCC, i.e., an agonist as described above, in combination with a pharmaceutically acceptable carrier, to thereby alleviate the abnormal condition. Alternatively, gene therapy may be employed to effect the endogenous production of hVRCC by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells in vivo and expression of the polypeptide in vivo. For overview of gene therapy, see Chapter 20, Gene Therapy and other Molecular Genetic-based Therapeutic Approaches, (and references cited therein) in Human Molecular Genetics, T. Strachan and A. P. Read, BIOS Scientific Publishers Ltd (1996).

### Formulation and Administration

Peptides, such as the soluble form of hVRCC polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex vivo, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

### Cloning the Human hVRCC cation Channel

The sequence of the hVRCC cation channel was first identified by searching a database containing approximately 2 million human ESTs, which was generated using high throughput automated DNA sequence analysis of randomly selected human cDNA clones (Adams, M.D. *et al., Nature 377*:3-174 (1995); Adams, M.D. *et al*., *Nature 355*:632-634 (1992); and Adams, M.D. *et al*., *Science 252*:1651-1656 (1991)). Sequence homology comparisons of each EST were performed against the GenBank database using the blastn and tblastn algorithms (Altschul, S.F. *et al., J. Mol. Biol*. *215*:403-410 (1990)). A specific homology search using the known rat VR1 amino acid sequence against this human EST database revealed one EST, from a macrophage cDNA library, with approximatively 60% similarity to VR1. The sequence comparison suggested that it contained the complete open reading frame of a new protein. Sequence of the gene was confirmed by double strand DNA sequencing using the TaqFs (Perkin Elmer) and the gene was shown to be completely new by a blast search against Genbank release 103. The entire hVRCC coding region containing the EcoRI-XhoI fragments was inserted into the expression vector bluescript (Stratagene).

### Example 2

### Cloning and Expression of hVRCC in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3 (Invitrogen). Mammalian host cells that could be used include, human HEK 293, H9 and Jurkat cells, mouse NIH3T3 and C127 cells, Cos 1, Cos 7 and mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al*., *Biochem. J. 227*:277-279 (1991); Bebbington *et al*., *Bio/Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pCMVsport3.0 contains the strong promoter (CMV) of the Cytomegalovirus. Multiple cloning sites, e.g., with the restriction enzyme cleavage sites EcoRI, XhoI, facilitate the cloning of the gene of interest.

### Example 3

### Tissue distribution of hVRCC mRNA expression

Northern blot analysis can be carried out to examine hVRCC gene expression in human tissues, using methods described by, among others, Sambrook *et al*., cited above. A cDNA probe containing the entire nucleotide sequence of the hVRCC protein can be labeled with ³²P using the Rediprime™ DNA labeling system (Amersham Life Science, Arlington, IL), according to manufacturer's instructions. After labeling, the probe can be purified using a CHROMA SPIN- 100™ column (Clontech Laboratories, Inc.), according to manufacturer's protocol number PT1200-1. The purified labeled probe was then used to examine various human tissues for hVRCC mRNA.

Multiple Tissue Northern (MTN) blots containing various human tissues can be obtained from Clontech and examined with the labeled probe using ExpressHyb™ hybridization solution (Clontech) according to manufacturer's protocol number PT1190-1. Following hybridization and washing, the blots can be mounted and exposed to film at -70°C overnight, and films developed according to standard procedures.

It will be clear that the invention may be practiced otherwise than as particularly described in the foregoing description and examples.

Numerous modifications and variations of the present invention are possible in light of the above teachings and, therefore, are within the scope of the appended claims.

The entire disclosure of all publications (including patents, patent applications, journal articles, laboratory manuals, books, or other documents) cited herein are incorporated by reference.

## Claims

1. An isolated polynucleotide comprising a nucleotide sequence that has at least 80% identity to a nucleotide sequence encoding the hVRCC polypeptide of SEQ ID NO:2 over its entire length; or a nucleotide sequence complementary to said nucleotide sequence.

2. The polynucleotide of claim 1 which is DNA or RNA.

3. A polynucleotide according to one of claims 1 and 2 wherein said nucleotide sequence is at least 80% identical to that contained in SEQ ID NO:1.

4. A polynucleotide according to one of claims 1 to 3 wherein said nucleotide sequence comprises the hVRCC polypeptide encoding sequence contained in SEQ ID NO:1.

5. A polynucleotide according to one of claims 1 to 4 which is polynucleotide of SEQ ID NO: 1.

6. An isolated hVRCC polynucleotide comprising a nucleotide sequence selected from the group consisting of :
(a) a nucleotide sequence having at least 80% identity to a nucleotide sequence encoding the hVRCC polypeptide expressd by the cDNA insert deposited at the ATCC with Deposit Number 209625 ; and
(b) a nucleotide sequence complementary to the nucleotide sequence of (a).

7. A DNA or RNA molecule comprising an expression system, wherein said expression system is capable of producing a hVRCC polypeptide comprising an amino acid sequence, which has at least 80% identity with the polypeptide of SEQ ID NO:2 when said expression system is present in a compatible host cell.

8. A host cell comprising the expression system of claim 7.

9. A process for producing a hVRCC polypeptide comprising culturing a host of claim 8 under conditions sufficient for the production of said polypeptide and recovering the polypeptide from the culture.

10. A process for producing a cell which produces a hVRCC polypeptide thereof comprising transforming or transfecting a host cell with the expression system of claim 7 such that the host cell, under appropriate culture conditions, produces a hVRCC polypeptide.

11. A hVRCC polypeptide comprising an amino acid sequence which is at least 80% identical to the amino acid sequence of SEQ ID NO:2 over its entire length.

12. The polypeptide of claim 11 which comprises the amino acid sequence of SEQ ID NO:2.

13. An antibody immunospecific for the hVRCC polypeptide of claim 11.

14. Use of (a) a therapeutically effective amount of an agonist of hVRCC polypeptide of claim 11 and/or (b) a polynucleotide according to one of claims 1 to 6 in a form so as to effect production of said hVRCC polypeptide activity *in vivo*, for the manufacture of a medicament for the treatment of a subject in need of enhanced activity or expression of hVRCC polypeptide.

15. Use of (a) a therapeutically effective amount of an antagonist of hVRCC polypeptide of claim 11 and/or (b) a nucleic acid molecule that inhibits the expression of the nucleotide sequence encoding said hVRCC polypeptide and/or (c) a therapeutically effective amount of a polypeptide that competes with said hVRCC polypeptide, for the manufacture of a medicament for the treatment of a subject having need to inhibit activity or expression of HVRCC polypeptide.

16. A process for diagnosing a disease or a susceptibility to a disease in a subject related to expression or activity of hVRCC polypeptide of claim 11 in a subject comprising:
(a) determining the presence or absence of a mutation in the nucleotide sequence encoding said hVRCC polypeptide in the genome of said subject; and/or
(b) analyzing for the presence or amount of the hVRCC polypeptide expression in a sample derived from said subject.

17. A method for identifying agonists to hVRCC polypeptide of claim 11 comprising:
(a) contacting cells produced by claim 10 with a candidate compound; and
(b) determining whether the candidate compound effects a signal generated by activation of the hVRCC polypeptide.

18. An agonist identified by the method of claim 17.

19. The method for identifying antagonists to hVRCC polypeptide of claim 11 comprising:
(a) contacting said cells produced by claim 10 expressing the hVRCC polypeptide on their surface with an agonist;
(b) contacting said cells with a candidate compound; and
(c) determining whether the signal generated by said agonist is diminished in the presence of the candidate compound.

20. An antagonist identified by the method of claim 19.
